# EUROPEAN PATENT APPLICATION

(11) **EP 0 776 669 A1**
(43) Date of publication of application: **04.06.1997**
(21) Application number: 95203247.2
(22) Date of filing: 27.11.1995
(51) Int. Cl.: A61L 2/26

(54) **Testpack for sterilizers**

(71) Applicant: MINNESOTA MINING AND MANUFACTURING COMPANY, St. Paul, Minnesota 55133-3427 (US)
(72) Inventor: Küpper, Anton, Hansastrasse 9, 41453 Neuss (DE); Hackler, Reiner, Hansastrasse 9, 41453 Neuss (DE); Kirk, Brian, Loughborough, Leicester LE11 1EP (GB); Shelley, Philip, Minnesota 55144-1000 (US)
(74) Representative: Hill, Cecilia Ann

(57) **Abstract**

A test pack, for use in testing the efficiency of a sterilization cycle in a sterilizer, includes a challenge medium (7) in the form of a particulate or sintered material. The challenge medium is contained in an enclosure (4), which is generally-conical in shape. The enclosure (4) has an opening (6) at the wide end for the entry of sterilant and a sterilant sensor (8) at the other end.

## Description

The present invention relates to test packs and testing systems for determining the efficacy of sterilization cycles in sterilizers.

A sterilization process used to sterilize medical and hospital equipment is only effective if a certain combination of environmental conditions is achieved within the sterilization chamber of the sterilizer. For example, when steam is used as a sterilant, the object of the sterilization process is to bring steam of a suitable quality, and at an appropriate temperature into contact with all surfaces of the articles being sterilized for a correct length of time

In some steam sterilizers the process of sterilization is typically conducted in three main phases. In the first phase, air trapped within the load being processed is removed. The second phase is a sterilizing stage, in which the load is subjected to steam under pressure for a recognised combination of time and temperature, which is known to effect sterilization. The third phase is a drying phase in which condensate formed during the first two phases is removed by evacuating the chamber.

Air removal from the sterilization chamber may be achieved in a number of ways. For example, in a gravity steam sterilizer, the principle of gravity displacement is utilized, in which steam entering at the top of the chamber displaces the air through a valve in the base of the chamber. In a prevacuum steam sterilizer, on the other hand, air is removed forcibly by deep evacuation of the chamber or by a combination of evacuation and steam injection at either subatmospheric and/or superatmospheric pressures.

Any air which is not removed from the sterilization chamber during the air removal phase of the cycle or which leaks into the chamber during a subatmospheric pressure stage due to faulty gaskets, valves or seals, may form air pockets within the load that is being sterilized. Likewise, any non-condensable gases (which, in this context, means gases having a boiling point below that of the sterilant) that are present in the sterilization chamber or are carried within steam supplied to the chamber may form gas pockets within the load. These air or gas pockets will create a barrier to steam penetration, thereby preventing adequate sterilizing conditions being achieved for all surfaces of the load. This is particularly true when porous materials such as hospital linens or fabrics are being sterilized since the air or gas pockets prohibit the steam from penetrating to the interior layers of such materials. As a result, sterilization may not occur. Therefore, there is a need to be able to determine the efficacy of sterilization cycles and in particular, to determine whether there has been sufficient steam penetration. Similarly, when a sterilant other than steam is used, there is a need to be able to determine that the sterilant has penetrated a load sufficiently for sterilization to take place.

One commonly-used procedure for evaluating the effectiveness of air removal during the air removal phase of a porous load steam sterilization cycle and/or for testing for the presence of non-condensable gases is known as the Bowie-Dick test. The typical Bowie-Dick test pack essentially consists of a stack of freshly laundered towels folded to a specific size, with a chemical indicator sheet placed in the centre of the pack. Chemical indicator test sheets undergo a visible change from one distinct colour to another, for example, from an initial white to a final black colour, upon exposure to the sterilization process. If the air removal within the sterilizer is insufficient, or if non-condensable gases are present during the process in sufficient quantity, an air/gas pocket will form in the centre of the pack thereby preventing steam from contacting the steam sensitive chemical indicator sheet. The consequence of inadequate steam penetration is a non-uniform colour development across the surface of the chemical indicator test sheet: thus, the presence of the air/gas pocket will be recorded by the failure of the indicator to undergo the complete or uniform colour change indicative of adequate steam penetration.

Biological indicators can also be used to provide information on the adequacy of a sterilization cycle. Biological indicator test systems typically employ living spores which are subjected to a sterilization cycle. After the cycle, the spores are incubated and the system detects if there is any growth. If there is no growth, it indicates that the sterilization process has been effective. Thus, biological indicators can determine whether conditions for sterilization were present, but the length of time to obtain results due to the incubation period is often at least 24 hours. Therefore, biological indicator systems are often used in conjunction with chemical indicators because the colour change of the chemical indictors provides an instant result. Further, by using both chemical and biological indicators, information on both the adequacy of the air removal stage and the sterilization stage is provided.

Parametric monitoring has also been used to either monitor or control a sterilization cycle to ensure proper sterilization conditions are attained. For example, in U.S. Patent No. 4,865,814 to Childress, an automatic sterilizer is disclosed which includes a microprocessor which monitors both the temperature and pressure levels inside the sterilization chamber and controls a heater to allow both pressure and temperature to reach predetermined levels before starting a timer. Once the timer is started, it is stopped if the pressure or temperature levels drop below a predetermined minimum. Since it is known that the pressure and temperature variables of saturated steam are dependent variables when saturated steam is enclosed in a sealed chamber, monitoring of these two variables can ensure that proper conditions are maintained during the sterilization cycle.

Although it is desirable to monitor environmental conditions within the sterilization chamber itself, it is generally considered more desirable to be able to monitor the environmental conditions within an actual load being sterilized or within a test pack (such as the Bowie-Dick test pack) that represents such a load. Although the typical Bowie-Dick test pack is generally recognized as adequate for use in determining the efficacy of the air removal stage of prevacuum sterilizers, it still presents many disadvantages. Since the test pack is not preassembled, it must be constructed every time the procedure is used to monitor sterilizer performance. The preparation, assembly and use of the towel pack is time consuming and cumbersome and, moreover, varying factors, such as laundering, prehumidification, towel thickness and wear, and the number of towels used, alter the test results. Therefore, alternative Bowie-Dick test packs have been developed to overcome these limitations.

An example of an alternative Bowie-Dick test pack for steam or gas sterilizers is described in EP-A-0419282. That test pack includes a container having top and bottom walls with a porous packing material disposed within the container. The packing material challenges the penetration of the sterilant by providing a restricted pathway which acts to impede the flow of the sterilant through the test pack. A removable lid seals the bottom end of the container, while a hole in the top wall of the container allows for the downward ingress of steam into the packing material within the container. The test pack includes a chemical and/or a biological indicator for detecting sterilant penetration. In the case of a chemical indicator, if sterilant successfully penetrates the packing material of the test pack, the chemical indicator sheet will undergo a complete colour change. If the sterilant does not sufficiently penetrate the packing material, the chemical indicator will not undergo a complete uniform colour change, thereby indicating inadequate air removal or the presence of non-condensable gas, or in other words, a Bowie-Dick test failure.

Other test packs for use in steam or gas sterilizers are described in EP-A-0 421 760; US-A-5 066 464; WO 93/21964 and US-A-5 270 217. In each of those test packs, sterilant from the sterilization chamber must cross some form of physical barrier before it reaches a sterilant sensor within the test pack.

Another known arrangement for challenging the penetration of sterilant to a particular location within a test pack comprises a very long (typically, 1.5m) stainless steel tube with a narrow bore (typically, 2.0 mm) which provides the only access for sterilant to the predetermined location. US-A-4 115 068 describes an indicator device which comprises an upright tube containing a temperature sensitive indicator strip. The tube is formed from a thermally insulating material and is lined with a thermally conducting material.

The problem with which the present invention is concerned is that of providing, for sterilizer testing systems, a test pack which is comparatively simple and inexpensive to manufacture but which will function reliably to enable ineffective sterilization cycles to be identified.

The present invention provides a test pack for use in determining the efficacy of a sterilization cycle in a sterilization chamber, the test pack comprising:
a housing made from a material which is impermeable to gas and liquid, the housing defining an enclosure having an opening for the entry of sterilant;
a sensor positioned to detect the presence of sterilant within the enclosure at a location remote from the opening; and
particulate material contained within the enclosure to challenge the penetration of sterilant to the said location. It is preferred, regardless of particle size, that the particulate material comprises at least 100 particles.

The present invention also provides a test pack for use in determining the efficacy of a sterilization cycle in a sterilization chamber, the test pack comprising:
a housing made from a material which is impermeable to gas and liquid, the housing defining an enclosure having an opening for the entry of sterilant;
a sensor positioned to detect the presence of sterilant within the enclosure at a location remote from the opening; and
sintered material contained within the enclosure to challenge the penetration of sterilant to the said location.

Preferably, the dimensions of the enclosure are such that the ratio of the length of the enclosure to the diameter at the wider end is within the range of from 0.5:1 to 3:1.

Preferably, the housing has a thermal conductivity within the range of from 0.15 to 1.00 W/m.°K, and a thermal capacity within the range of from 0.5 to 2.5 kJ/kg.°K. The housing may comprise a polymeric material, for example polysulfone; polyphenylsulfone; polyethersulfone; polyetheretherketone (PEEK); polytetrafluorethylene; poly(etherketoneetherketoneketone) (PEKEKK); or polymethylpentene.

The present invention further provides a test pack for use in determining the efficacy of a sterilization cycle in a sterilization chamber, the test pack comprising;
a housing made from a material which is impermeable to gas and liquid;
a generally-conical enclosure within the housing;
an opening for the entry of sterilant in the wider end of the enclosure, and a sensor positioned to detect the presence of sterilant within the enclosure at the narrower end thereof; and
a challenge medium contained within the enclosure to challenge the penetration of sterilant to the narrower end of the enclosure,
wherein the housing has a thermal conductivity within the range of from 0.15 to 1.00 W/m.°K and a thermal capacity within the range of from 0.5 to 2.5kJ/kg.°K, and the dimensions of the enclosure are such that the ratio for the length of the enclosure to the diameter of the wider end is within the range of from 0.5:1 to 3:1. The housing material may be a polymeric material. Preferably, the housing material is polysulfone; polyphenylsulfone; polyethersulfone; polyetheretherketone (PEEK); polytetrafluorethylene; poly(etherketoneetherketoneketone) (PEKEKK); or polymethylpentene.

In a test pack in accordance with any aspect of the present invention, the sensor may be a temperature sensor positioned to measure the temperature of the said location. Alternatively, the sensor may be a biological or a chemical indicator.

By way of example only, embodiments of the invention will now be described with reference to the accompanying drawings, in which:
Fig. 1 is a perspective view of a test pack in accordance with the invention;
Fig. 2 shows a longitudinal cross-section of the device of Fig. 1;
Figs. 3 to 6 show diagrammatic cross-sections of alternative test pack housings; and
Figs. 7 and 8 show diagrammatic cross-sections of other test packs in accordance with the invention.

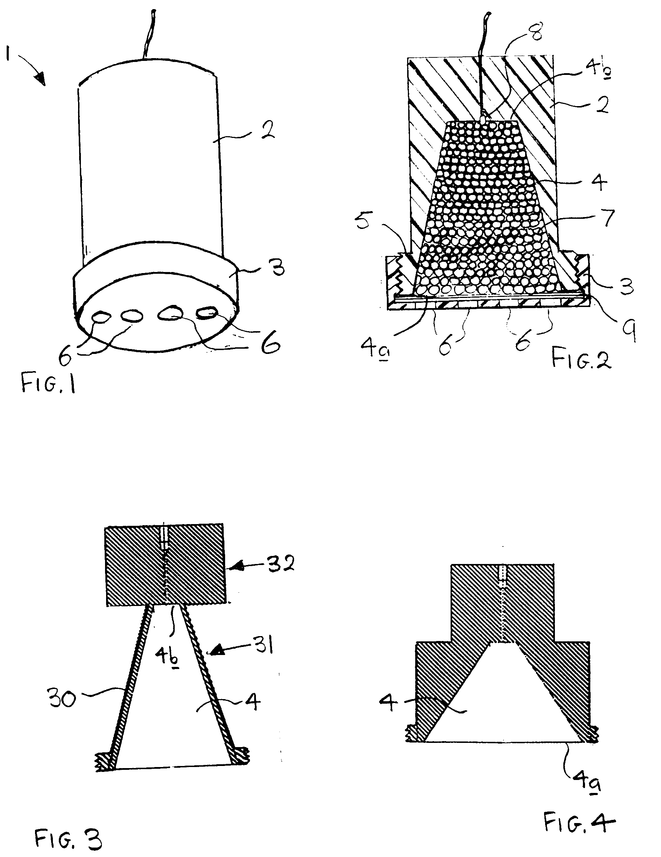

Figs. 1 and 2 show a test pack 1 suitable for use in a system for testing the efficacy of a sterilization cycle, either in a steam sterilizer, or in a low temperature gas sterilizer in which sterilization is carried out using a microbiocidal agent (for example, formaldehyde) in the presence of moisture. The test pack 1 is intended to be located in the sterilization chamber of the sterilizer to provide a challenge path along which sterilant (for example, steam) from within the chamber must pass before it can be detected by a sensor at a predetermined location within the device. If the presence of sterilant at the predetermined location is not detected by the sensor during a sterilization cycle (indicating that the conditions within the sterilization chamber have not enabled sterilant to penetrate the challenge path), the sterilization cycle is judged to be ineffective.

The test pack 1 shown in Figs. 1 and 2 comprises a hollow housing formed by an open-ended body portion 2 which is closed by an end cap 3. The body portion 2 defines an enclosure 4 of generally conical shape, the wide end 4a of which is the open end of the body portion. Externally, the body portion 2 has the form of a cylinder with a threaded annular flange 5 at the open end, to which the end cap 3 is secured. The body portion 2 and the end cap 3 are formed from a material which is impermeable to gas and liquid and has selected thermal characteristics, described below.

The end cap 3 is formed with apertures 6 through which sterilant can enter the enclosure 4 during a sterilization cycle as described above. The enclosure 4 is at least partially filled with a particulate challenge medium 7, described in greater detail below, the function of which is to challenge the penetration of sterilant into the enclosure during the sterilization cycle. A sterilant sensor 8, of any suitable type, is located at the narrow, closed, end 4b of the enclosure 4.

The test pack 1 is preferably used in the orientation shown in Figs. 1 and 2 that is, with the end cap 3 at the bottom. To prevent the escape of particulate 7 through the apertures 6, one or more layers of a retaining material 9 are placed over the open end of the enclosure 4. The retaining material should, of course, not prevent sterilant from entering the enclosure 4 through the apertures 6.

When the test pack 1 is subjected to a sterilization cycle within a sterilization chamber, the particulate 7 impedes the flow of sterilant into the enclosure 4 so that pockets of air/non-condensable gases will tend to remain within the enclosure, particularly towards the closed end 4b. The size of the air/gas pockets is indicative of the efficiency of the sterilization cycle, being larger when the air removal phase at the cycle is less adequate. By an appropriate selection of the particulate 7, and of the thermal properties of the test pack housing 2, 3, it can be arranged that sterilant will not penetrate, or not penetrate fully, to the narrow end 4b of the enclosure 4 when the environmental conditions in the sterilization chamber do not satisfy the requirements for effective sterilization. Detection of the presence of sterilant by the sensor 8 is then an indication that a sterilization cycle has been effective, while non-detection (or partial detection) is an indication that a sterilization cycle has failed to meet requirements.

The test pack 1 can be of any suitable size and, preferably, is as small as possible. Typically, the length of the enclosure 4 (i.e. from the wide, open, end 4a to the narrow, closed, end 4b) is within the range 3 to 30 cm and the diameter of the wide end is within the range 2 to 15 cm although dimensions outside those ranges are not excluded. The length/diameter ratio is preferably in the range 0.5/1 to 3/1 and, for the test pack shown in Figs 1 and 2, is about 1/1. The material from which the body portion 2 is formed should have a low thermal conductivity in combination with a thermal capacity which results in some condensation of moisture within the enclosure 4 during a sterilization cycle, leading to the entrapment, within the enclosure of non-condensable gases such as air. On the other hand, the thermal capacity of the body portion 2 should not be so high that the particulate 7 becomes clogged with moisture. Preferably, the material from which the body portion 2 is formed has a thermal conductivity in the range 0.15 to 1.00W/m.°K and a thermal capacity in the range 0.5 to 2.5 kJ/kg.°K. Suitable materials for the body portion include polysulfone, polyphenylsulfone, polyethersulfone, polyetheretherketone (PEEK), poly(etherketoneetherketoneketone) (PEEKEK), polytetrafluorethylene and polymethylpentene.

The particulate 7 within the enclosure 4 should be selected to ensure that effective sterilization cycles can be distinguished from non-effective cycles in a reproducible and predictable manner. The features of the particulate which enable that to be accomplished include the nature and amount of the material, the particle size, and the range of particle sizes. The particulate may be a glass, a ceramic, or a polymer, or combinations thereof.

The enclosure 4 need not be filled completely by the particulate: depending on the particulate it can, for example, be only 50% full in which case some form of porous barrier is required within the enclosure to contain the particulate at the narrower end, adjacent the sensor 8. The particle size may be as small as 7µm or as large as 5 mm, but when various sizes are present they preferably do not differ by more than 20% from the average particle size. Regardless of the particle size, there are preferably at least 100 particles in the enclosure 4. The particles may be either solid or hollow.

Suitable particulates include:
(i) solid glass beads or spheres (for example, 46µm beads and 7.8µm beads both available, under the trade designations "Zeeospheres, 850" and "Zeeospheres, 600", from 3M Zeelan Industries, of St. Paul, Minnesota, U.S.A., and 4 mm spheres available, under the trade designation "G0300/53", from Fisher Scientific of Loughborough, Leicestershire, England);
(ii) hollow ceramic spheres having a diameter within the range 50µm to 4 mm (for example, 150-300µm hollow spheres available, under the trade designation "Z-Light", from 3M Zeelan Industries, of St. Paul, Minnesota, U.S.A);
(iii) polypropylene granules (for example, 4 mm granules available, under the trade designation "Novolen 1142 N", from BASF of Ludwigshaven, Germany).

A preferred particulate comprises hollow ceramic spheres as in (ii) above, selected so that the sizes of the spheres do not differ by more than 20% from the average size.

The retaining material 9 may be spun bond polypropylene or any similar material.

Although the enclosure 4 of the test pack 1 has a generally-conical form, it need not have linear sides but could, for example, have stepped or curved sides while still being generally conical. Moreover, although the generally-conical form is preferred, the enclosure could have any other suitable shape: it could, for example, have some other tapering shape such as pyramidal, or it could be cylindrical.

Some alternative shapes for the body portion 2 of the test pack are shown in Figs. 3 to 6.

The body portion shown in Fig. 3 defines an enclosure 4 of the same shape as that of Fig. 2 but, in this case, the side walls 30 of the enclosure are of constant thickness so that the body portion comprises a conical portion 31 and, at the narrow end 4b of the enclosure, a cylindrical portion 32 corresponding to the upper end of the body portion 2 in Fig 2.

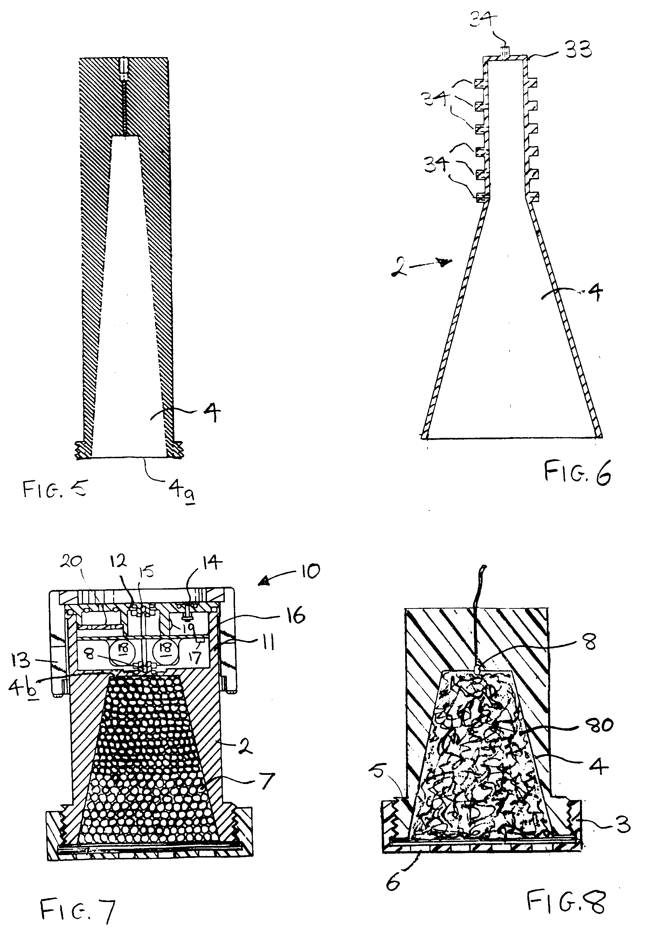

The body portion shown in Fig. 4 defines an enclosure 4 which is also generally conical but shorter than that of Fig. 2 and with a larger diameter open end 4a so that the length/diameter ratio in this case is about 0.5/1. The body portion shown in Fig. 5, on the other hand, defines a conical enclosure which is longer than that of Fig. 2 and has a smaller diameter open end 4a so that the length/diameter ratio is about 3/1.

Fig. 6 shows a body portion 2 which is similar to that shown in Fig. 3 except that the cylindrical upper end 32 is replaced by a tubular portion 33 which opens at one end into the enclosure 4 and is closed at the other end. A plurality of sterilant sensors 34, one at the closed end of the tubular portion 33 and the others spaced along the length of the tubular portion, replace the single sensor 8 of Fig 3.

Each of the alternative body portions illustrated in Figs. 3 to 6 preferably has similar thermal characteristics to that of Figs. 1 and 2, and may be formed from similar materials.

The sensors 8, 34 of Figs. 2 to 6 are temperature sensors, connected to provide an electrical signal indicative of the temperature in the adjacent region of the enclosure 4. When such a test pack is subjected to a sterilization cycle, the particulate 7 prevents the sensor(s) 8, 34 from being exposed to the full effect of the sterilant in the chamber, giving rise to a difference between the temperature sensed by the sensor and the environmental temperature in the chamber. Detection of that temperature difference enables the efficacy of the sterilization cycle to be determined.

It is, however, not essential for temperature sensors to be employed in the test packs described above with reference to Figs 1 to 6. Sensors which detect a different environmental parameter, for example humidity, could be used instead. Alternatively, the upper part of the body portion 2 of a test pack could be modified to incorporate a chemical or a biological indicator to detect the presence of sterilant at the closed end of the enclosure 4. If required, several sensors could be employed. For example, a chemical indicator could be used in combination with a biological indicator, or sensors could be used to detect several environmental parameters (e. g. temperature, humidity and pressure).

Fig. 7 illustrates an electronic test unit 10 which is a development of the test pack shown in Figs. 1 and 2. The test unit 10 is a self-contained unit which can be placed in a sterilization chamber to determine the efficacy of a sterilization cycle. As described below, the test unit 10 functions, during a sterilization cycle, to measure the temperature at two locations, one being at the narrow end of the enclosure 4 and the other being at a reference point within the sterilization chamber itself. Those temperature measurements are then used to determine whether or not the sterilization cycle, in particular the air removal phase of the cycle, was effective (i.e. met certain prescribed requirements).

In the test unit shown in Fig. 7, the end wall of the body portion 2 is hollowed-out to form a housing 11 which contains the electronic components of the test unit. Those components will be described below. The electronics housing 11 has a removable end cap 12 and is positioned within an outer housing 13 to which it secured, for example by screws. When secured, the outer housing 13 holds the end cap 12 to the electronics housing 11 so that the latter is sealed. Outer housing 13 is constructed of a structurally rigid material, such that when stressed, it returns to its original shape. For example, any type of metal, as well as glass fiber or carbon fiber reinforced plastic with softening temperatures higher than 150°C can be used for outer housing 13.

The components housed inside electronics housing 11 may be protected from the extreme heat within the sterilization chamber by a vacuum within the housing. To that end, electronics housing 11 includes one-way valve 14 which opens when the pressure external to the housing 11 falls below a predetermined value. Then, when a vacuum is pulled within a sterilization chamber with test unit 10 placed inside, valve 14 opens to allow a vacuum also to be pulled within electronics housing 11. Electronics housing 11 contains the temperature sensor 8 together with a second temperature sensor 15. Temperature sensors 8 and 15 may be any suitable type of temperature transducer, for example, thermocouples or thermistors. Temperature sensor 8 as already described with reference to Fig. 2 is positioned such that it measures the temperature at the end 4b of the enclosure 4. Temperature sensor 15, on the other hand, measures the external temperature. Thus, when unit 10 is placed within a sterilization chamber, temperature sensor 15 measures the chamber temperature.

Housing 11 also contains a circuit board 16, mounted so that it is thermally isolated from the walls of the housing to prevent conduction of external heat to the electronics mounted on the board, which include a microprocessor and a memory, preferably an electrically erasable programmable read-only memory (EEPROM). Surface mounted chips 17, batteries 18, the temperature sensors 8 and 15, a light emitting diode 19 and a pressure sensor 20 are all electrically connected to circuit board 16.

As temperature sensors 8 and 15 measure temperatures, the temperature readings are stored in the test pack memory together with time data from the microprocessor. Once the microprocessor determines that a sterilization cycle is complete, it then determines (from the stored temperature readings) whether the sterilization cycle is satisfactory, in other words, that the sterilant has adequately penetrated the enclosure 4 of the test unit 10.

If the microprocessor determines that the sterilization cycle was satisfactory, light emitting diode (LED) 19 emits light. In a completely self-contained electronic test pack, only a single LED is necessary to indicate whether the cycle has passed. With a single LED, the LED may continuously burn to indicate a pass cycle and may flash to indicate a fail cycle. Alternatively, two LEDs may be used, to indicate a pass cycle and fail cycle respectively. If the sterilization cycle has passed, one LED emits a green light. If the microprocessor determines that the sterilization cycle has failed, the other LED emits a red light.

In some situations, it is desirable to transfer the data stored in the memory of the unit to an outside processor or memory or a printer. Data transfer may be initiated by actuating a magnetically actuated switch (not shown), preferably a reed switch.

The manner in which the unit 10 determines the efficiency of a sterilization cycle is, briefly, as follows. As already described with reference to Figure 1, the challenge medium 7 prevents the sensor 8 from being exposed to the full effects of sterilant, thus giving rise to a difference between the temperature at the sensor 8 and the temperature at the sensor 15. The unit 10 determines if that temperature difference exceeds a predetermined value at a predetermined point within the sterilization cycle and, if so, the cycle is judged to be unsatisfactory. This predetermined temperature difference is determined by validation experiments in which the performance of the electronic test unit 10 is compared with that of a standard Bowie-Dick textile test pack according to recognized International, European or National standards. For example, the test unit 10 could be pre-programmed so that, if the temperature difference is greater than 2°C in a 2 minute and 40 seconds period after the chamber temperature reaches a sterilization hold temperature of 134°C, the cycle is considered unsatisfactory. Further, the chamber temperature must remain above an adequate sterilization temperature for sterilization to occur.

While the examination of the temperature difference between the external and internal temperature (as just described) provides direct information on the penetration of heat to the sensor 8 located within the test unit 10, it does not directly reflect penetration of sterilant to the sensor (although, by inference, rapid equilibrium between the sensing point within the enclosure 4 and the sterilization chamber indicates the absence of an insulating air/gas pocket in the enclosure). In the case of a steam sterilizer, it is possible, however, to measure directly the moisture penetration to the sensing point within the challenge device. To that end, a moisture sensor, such as a conductivity sensor or a relative humidity sensor, can be used instead of or in addition to, the temperature sensor 8 to determine adequate moisture penetration to the sensing point within the challenge device and therefore, by inference, steam. The temperature sensor 15 measuring the sterilization chamber temperature remains the same.

The test unit shown in Fig. 7 can be modified to incorporate a chemical or biological indicator, rather than the temperature sensors 8, 15 and associated electronics. The indicator is located in the housing 11 so that it is exposed to the conditions at the narrow end 4b of the enclosure 4. To that end, the end wall of the enclosure 4 is apertured to permit the passage of sterilant into the housing 11 and covered with a porous barrier to prevent the particulate 7 from entering the housing 11. Biological and chemical indicators are both well known: a suitable biological indicator is available, under the trade designation "ATTEST", from Minnesota Mining and Manufacturing Company of St. Paul, Minnesota, U.S.A., and a suitable chemical indicator is available, under the trade designation "Comply 1250", also from Minnesota Mining and Manufacturing Company of St. Paul, Minnesota, U.S.A.. The indicator, whether biological or chemical, is located in the housing 11 of the test unit prior to a test of a sterilization cycle and is removed when the cycle has been completed so that it can be examined to determine whether or not the cycle was effective. A replacement indicator is then put into the housing 11 of the test unit so that the unit can be re-used.

Fig 8 shows another test pack, similar to that shown in Figs. 1 and 2 except that the particulate 7 is replaced by a porous plug 80 of sintered plastics or ceramic material, for example a sintered HD (high density) polyethylene material such as that available, under the trade designation "SIPERM HP", from Thyssen Magnettechnik GmbH of Dortmund, Germany. The porous plug 80 is preformed by sintering (i.e. heating particulate material to cause it to coalesce into a solid) in a mould having the same dimensions as the enclosure 4 of the test pack. More specifically, the particulate material for the plug 80 is mixed with a binder material which is volatile or easily melted. The mixture is then injected into, or placed in, the mould and sintered. During the sintering process, the binder material evaporates or melts rapidly, leaving a porous plug. Alternatively, it is possible to use other binder materials which are not removed during the sintering process but are removed after sintering (for example, using chemical solvents) to leave a porous plug.

The porosity of the plug 80, produced by the sintering process, can be varied by changing the shape, size and distributions of the particles in the mixture that is loaded into the mould.

It is not essential for the plug 80 to fill the entire length of the enclosure 4 as shown in Fig. 8 although it should always contact the walls of the enclosure and be located at the narrower end, adjacent the sensor 8. In that case, the plug would be formed in a shorter mould.

It will be appreciated that a challenge medium formed from a sintered material as shown in Fig. 8 could be used in any of the body portions shown in Figs. 3 to 6 or in the test unit shown in Fig. 7. Moreover, since it is possible to produce plugs of sintered materials in other shapes, the use of sintered material as a challenge medium is not restricted to test packs, such as those shown in Figs. 1 to 8, in which a conical plug is required. It would, for example, be possible to use cylindrical plugs of sintered material as a challenge medium in test packs of the shape described in EP-A-0 419 282.

The challenge media described above for the test packs/units shown in Figs. 1 to 8 are intended to be re-usable (i.e. the test packs/units would not be disposed of after a single use). It will be appreciated, however, that both the particulate material shown in Figs. 2 and 7 and the sintered material shown in Fig. 8 could be replaced when necessary, although the rest of the test pack/unit would be retained and re-used. Alternatively, both forms of challenge medium could be used in disposable test packs of the type described in EP-A-0 419 282.

It will also be appreciated that the use of particulate or sintered material as the challenge medium in the test packs shown in Figs. 1 to 8, although preferred, is not essential. Other forms of challenge medium, for example a foam material or any of the packing materials described in the above-mentioned EP-A-0 419 282, could be used in a test pack having the construction and thermal characteristics described above with reference to Figs. 1 to 8 (i.e. a test pack in which the challenge medium is contained within a generally conical enclosure formed in a housing having a thermal conductivity in the range 0.15 to 1.00W/m.°K and a thermal capacity in the range 0.5 to 2.5 kJ/kg. °K, with the dimensions of the enclosure being such that the ratio of the length of the enclosure to the diameter of its wider end is in the range 0.5:1 to 3:1).

## Claims

1. A test pack for use in determining the efficacy of a sterilization cycle in a sterilization chamber, the test pack comprising:
a housing made from a material which is impermeable to gas and liquid, the housing defining an enclosure having an opening for the entry of sterilant;
a sensor positioned to detect the presence of sterilant within the enclosure at a location remote from the opening; and
particulate material contained within the enclosure to challenge the penetration of sterilant to the said location.

2. A test pack as claimed in claim 1 wherein the particulate includes hollow particles.

3. A test pack as claimed in claim 1 or claim 2, wherein the particulate is a glass, or a ceramic, or a polymer, or combinations thereof.

4. A test pack as claimed in any one of the preceding claims, wherein the particulate comprises hollow ceramic spheres having a diameter within the range of from 50µ to 4mm, preferably 150 to 300µm.

5. A test pack for use in determining the efficacy of a sterilization cycle in a sterilization chamber, the test pack comprising:
a housing made from a material which is impermeable to gas and liquid, the housing defining an enclosure having an opening for the entry of sterilant;
a sensor positioned to detect the presence of sterilant within the enclosure at a location remote from the opening; and
sintered material contained within the enclosure to challenge the penetration of sterilant to the said location.

6. A test pack as claimed in claim 6, in which the sintered material is in the form of a plug which is shaped to fit into the enclosure, adjacent the sensor.

7. A test pack as claimed in claim 5 or claim 6, in which the sintered material is a plastics or a ceramic material.

8. A test pack as claimed in any one of the preceding claims, wherein the enclosure is generally-conical and the opening for the entry of sterilant is in the wider end of the enclosure, the sensor being positioned to detect the presence of sterilant at the narrower end of the enclosure.

9. A test pack for use in determining the efficacy of a sterilization cycle in a sterilization chamber, the test pack comprising:
a housing made from a material which is impermeable to gas and liquid;
a generally-conical enclosure within the housing;
an opening for the entry of sterilant in the wider end of the enclosure, and a sensor positioned to detect the presence of sterilant within the enclosure at the narrower end thereof; and
a challenge medium contained within the enclosure to challenge the penetration of sterilant to the narrower end of the enclosure,
wherein the housing has a thermal conductivity within the range of from 0.15 to 1.00 W/m.°K and a thermal capacity within the range of from 0.5 to 2.5 kJ/kg.°K, and the dimensions of the enclosure are such that the ratio of the length of the enclosure to the diameter of the wider end is within the range of from 0.5:1 to 3:1.

10. A test pack as claimed in claim 9, wherein the housing material is a polymeric material, for example polysulfone; polyphenylsulfone; polyethersulfone; polyetheretherketone (PEEK); polytetrafluorethylene; poly(etherketoneetherketoneketone) (PEKEKK); or polymethylpentene.

11. A test pack as claimed in any one of the preceding claims, wherein the sensor is a temperature sensor positioned to measure the temperature at the said location.

12. A sterilizer testing system for determining the efficacy of a sterilization cycle in a sterilization chamber, the system comprising a test pack as claimed in claim 11 in combination with a sensor for measuring the environmental temperature within the sterilization chamber.
